# EUROPEAN PATENT APPLICATION

(11) **EP 1 417 943 A1**
(43) Date of publication of application: **12.05.2004**
(21) Application number: 04001016.7
(22) Date of filing: 19.04.2002
(51) Int. Cl.: A61F 9/008, A61F 9/01

(54) **METHOD FOR PHOTODISRUPTION OF THE EYE**

(30) Priority: 19.04.2001 US 285138 P
(62) Divisional of application: 02764253.7
(71) Applicant: Intralase Corp., Irvine, CA 92618 (US)
(72) Inventor: The designation of the inventor has not yet been filed
(74) Representative: Grünecker, Kinkeldey, Stockmair & Schwanhäusser Anwaltssozietät

(57) **Abstract**

A method for control of a laser system for photodisruption of a target, said method comprising the steps of: directing a laser beam of said laser system to perform a horizontal resection procedure based upon predetermined pattern parameters; displaying an image of the target within a display device; wherein said predetermined pattern parameters include a variable Z-radius setting.

## Description

### CROSS-REFERENCE TO RELATED APPLICATIONS

This application claims the benefit of the filing date of Provisional Application Serial No. 60/285,138 filed April 19, 2001, now pending (hereby incorporated by reference for all purposes).

### BACKGROUND AND SUMMARY OF THE INVENTION

The present invention relates generally to a method, system and/or apparatus for controlling and managing laser photodisruption procedures. In particular, the present invention relates to a method, system and/or apparatus for the computer-based control, management, and performance of photodisruption procedures on bodily tissue. In one application, instrument software is implemented on a computer-based laser system to perform procedures for photodisruption of tissue of a patient's eye. Based on preprogrammed and selectable parameters, the instrument software controls a laser source and focusing assembly to photo disrupt corneal tissue whereby substantially horizontal and/or vertical resections of the corneal tissue are created.

The instrument software controls the system startup, calibration, laser firing during a procedure, and provides a user interface to the operator of the laser system. The instrument software preferably uses a real-time, multi-tasking operating system such as QNX with a graphical user interface (for example Photon). The instrument software's main tasks include the following: operator interface, pattern parameter setup, controlling the laser beam pattern during a procedure, instrument protective controls and error management, instrument setup functions, and miscellaneous user account management functions.

The laser system utilizes a laser source for generating a laser beam for the photodisruption of tissue of the eye and an optical delivery system for focusing the laser beam. A computer processing unit executes instructions from instrument software. The instrument software is configured to control the operation of the laser source and optical delivery system. A monitor is coupled with the computer processing unit for providing a visual interface of the instrument software to an operator of said laser system. A video camera is utilized for obtaining images of a patient's eye which are displayed via a graphical user interface of the instrument software. Input devices are coupled with the computer processing unit for receiving commands to move the optical delivery system.

The instrument software provides a computer-implemented method for control of the laser system. Among various functionalities, the instrument software provides management and security for users, registration and maintenance of patient data, performance and management of photodisruption procedures, and monitoring of system errors.

The instrument software manages the procedure for photodisruption of tissue of the eye to create a substantially horizontal resection, for example, for the creation of a flap of corneal tissue or for creation of a ring or tunnel configuration in the corneal stroma. An image from a live video feed of a patient's eye is displayed within a graphical user interface. For creation of a flap, the operator of the laser system is prompted with a selection of flap pattern parameters. The parameters are initially set to default values, but may be changed by the user. Utilizing the selected flap pattern parameters, the instrument software directs the laser beam to perform a photodisruption procedure. The end result is a controlled, precise definition of a horizontal resection. Some of the flap pattern parameters are used by the instrument software include: upper diameter, depth in cornea, hinge angle, hinge position, and normal/inversed flap indicator.

The instrument software provides a unique benefit of defining horizontal resections utilizing different patterns along which to direct the laser beam. For example, one horizontal resection is defined by the instrument software directing the laser beam to photodisrupt tissue beginning at the visual center of a patient's eye and proceed in a circular path about the visual center and continuing directing the laser beam to photodisrupt tissue in concentric circles until a desired horizontal resection diameter is reached.

Another horizontal resection is formed by the instrument software by directing the laser beam to photodisrupt tissue beginning at the visual center of a patient's eye and proceeding in spiral path about the visual center extending outwardly until a desired horizontal resection diameter is reached.

Similar to the creation of a horizontal resection, the instrument software also controls the photodisruption of the tissue to create a vertical resection. This vertical resection, which may be at an angle other then 90 degrees relative to the plane of the horizontal resection, is used for creating a side cut for a flap of tissue or an entry cut for a ring configuration.

The instrument software also provides for the selection of preprogrammed ring pattern parameters for the creation of a horizontal resection in the shape of a planar O-ring. Utilizing the selected ring pattern parameters, the instrument software directs the laser beam to photodisrupt tissue to create a ring or tunnel resection. Some of the ring pattern parameters include: inner diameter, outer diameter, cornea thickness, and incision axis.

of tissue of. Some of the ring pattern parameters include: inner diameter, outer diameter, cornea thickness, and incision axis. Utilizing the selected ring pattern parameters, the instrument software directs the laser beam to photodisrupt tissue to create a vertical resection.

One aspect of the instrument software is the management of error conditions to provide a high degree of safety while performing a photodisruption procedure. The instrument software monitors all system errors during startup, idle state, and during the procedure and takes appropriate actions depending on the severity level of the error.

Another feature of the instrument software is secured access and operation of certain functions of the instrument software. Each user is granted access to functionality of the instrument software based on their pass-level. In one embodiment, four different passlevels (User, Owner, Maintenance, and R&D) are utilized. The pass-level model provides for a secure access to functions and operations of the laser system to qualified personnel.

The instrument software consists of several processes (programs) running concurrently and communicating with each other. Each process has a unique identification number and accomplishes a specific, and very often limited, task. Two general types of processes exist: servers and clients. A client sends a request to a server for a service and waits for a reply from the server. After the service is provided, the server sends back to the client a reply message. Some processes run as long as the instrument software is running. Other processes have a limited duration. Processes may be generated and terminated by other processes.

The instrument software may be run in different modes: wet, demo and dry. The wet mode is the normal operating mode. This mode takes full advantage of the input/output capabilities of the instrument software and instrument devices.

While in the demo mode, the instrument software allows simulated use of the instrument software without the necessity or operation of the instrument devices. The demo mode may be initiated using a demo option upon invocation of the instrument software. The demo option does not send any commands to or expect input from the instruments devices. This mode allows the instrument software to run for simulation purposes on a stand-alone computer, such as a PC.

While in the dry mode, the instrument software allows operation of the instrument software and instrument hardware, but will not fire the laser. The dry mode allows sequencing of the instrument without turning on the laser. This mode may be used during instrument debugging and testing.

The foregoing has outlined rather broadly the features and technical advantages of the present invention in order that the detailed description of the invention that follows may be better understood. Additional features and advantages of the invention are described hereinafter which form the subject of the claims of the invention. It should be appreciated by those skilled in the art that the conception and specific embodiment disclosed may be readily utilized as a basis for modifying or designing other structures for carrying out the same purposes of the present invention. It should also be realized by those skilled in the art that such equivalent constructions do not depart from the spirit and scope of the invention as set forth in the appended claims. The novel features which are believed to be characteristic of the invention, both as to its organization and method of operation, together with further objects and advantages are better understood from the following description when considered in connection with the accompanying figures. It is to be expressly understood, however, that each of the figures is provided for the purpose of illustration and description only and is not intended as a definition of the limits of the present invention.

### BRIEF DESCRIPTION OF THE DRAWINGS

For a more complete understanding of the present invention, reference is now made to the following descriptions taken in conjunction with the accompanying drawing, in which:

FIG. 1 is a software context diagram describing the interfaces between the instrument software and hardware for the laser unit;

FIG. 2a is a navigational model illustrating how a user interacts with the instrument software to access functionality and information;

FIG. 2b is a navigation model illustrating maintenance and R&D user interaction;

FIG. 3 is an instrument process context diagram; and

FIG. 4 is a top level process communication hierarchy.

### DETAILED DESCRIPTION OF THE INVENTION

### Instrument Hardware

In one embodiment, the laser system is used for photodisruption of tissue of the eye, such as the corneal stroma. The system utilizes instrument software to control a laser beam of constant energy, composed of an optical train of pulses with a duration of approximately 600 femtoseconds at a repetition rate of up to 10,000 Hz. The actual energy in the emitted pulse train is determined by rotating a variable attenuator, an energy attenuation wheel, which operates under stepper motor control.

Energy measuring devices are placed in the optical path between the attenuator and safety shutters. These devices measure a constant fraction of the energy transmitted to the optical delivery system.

The optical delivery system consists of an articulated arm, Z-axis lift, X-axis and Y-axis translation, X, Y, Z-axis galvanometer scanners, telescope, Z-axis focusing lens, turning mirror, microscope and a disposable lens that makes the contact with the patient's eye.

The optical delivery system determines a 3-dimensional position in the patient's cornea at which the laser is focused. When the laser is pulsed, the energy delivered to the point of focus is sufficient to ionize tissue (photodisrupt) in a very small volume. A plane of photodisrupted tissue is created by the process of repetitively setting a focal point and pulsing the laser. A moving mirror is used to maintain beam position/alignment to the delivery system. The beam steering system compensates for any beam movement due to laser bench drift and/or articulating arm movement. The beam steering system maintains the beam within certain tolerance of the original beam alignment on target. The system performs the procedure after the energy wheel calibration (startup) and before laser firing. The system is comprised of a moving mirror assembly, quadrature detector photodiode and a solenoid shutter assembly.

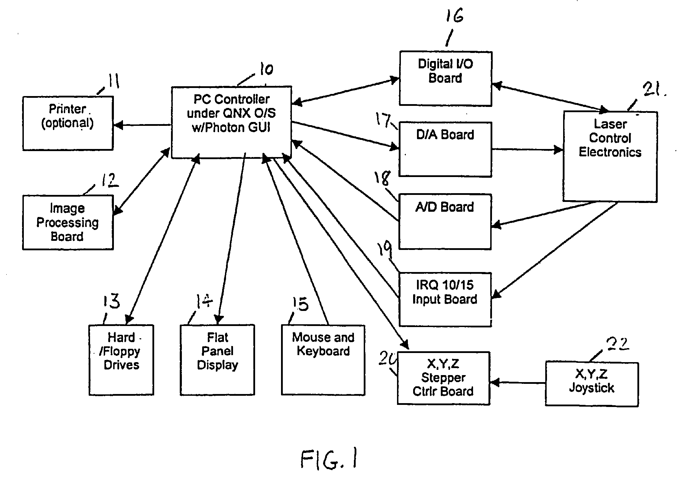

Now referring to Figure 1, a software context diagram illustrates interfaces between the instrument software and hardware for the laser system. A floppy drive and hard drive 13 are connected to the CPU 10. The floppy disk drive is used to load program and data files onto the laser system and to backup data such as user files. Hard drives are located within the laser system and store the operating system software. In one embodiment, the QNX™ 4.25 operating system is used with the laser system. In addition to storing the operating system, other software may be stored on the hard drive(s).

A flat panel display 14 is preferably used for graphically displaying information from the instrument software. The display 14 provides a visual interface between the laser system and the operator. A keyboard and mouse (or trackball) 15 is used by the operator to interact with the laser system. The operator inputs commands and data using the keyboard to interact with the instrument software. The mouse (or trackball) 15 is used by the operator to move a cursor on the screen of the display and select options presented by the instrument software. A joystick 22 is used by the operator to control the stage X, Y, Z stepper motors.

A digital I/O board 16 is used to control discrete signals that turn on and off devices in the laser system. Also, the board 16 is used to monitor various sensors that are located in the laser system which are then used to provide information to the instrument software.

A digital to analog board 17 and analog to digital board 18 are used by the laser system to control all system analog inputs and outputs. A stepper controller board 20 is used to control the X,Y,Z stage movement. An image processing board 12 is used to perform the laser beam to pupil alignment procedure. A vision machine is used to center the laser beam to the desired location in the pupil before performing the procedure. Preferably, a Watec™ Digital Color CCD camera, model LCL-307HS (NTSC interface) and Matrox Genesis™ display/image acquisition board are used.

An interrupt request board 19 is used to generate interrupt requests. In one embodiment, the interrupts '10' and '15' are used. Interrupt '10', based on the laser firing repetition rate causes the instrument software to output a new set of X, Y, Z values to the galvanometer control digital/analog board. Interrupt '15', generated when an error occurs in the instrument, causes exception events based on the severity of the error.

Additionally, a variable focal length objective is used to combine two functionalities in a single design. One function is during a surgical procedure to focus the laser light into the tissue for photodisruption. The other function is to provide the operating doctor a long focal length surgical microscope. The distance from the eye is increased to approximately 6 inches. Sensors and lens motor control hardware signals are directly connected by I/O wire to digital I/O boards.

### Instrument Software - Functional Architecture

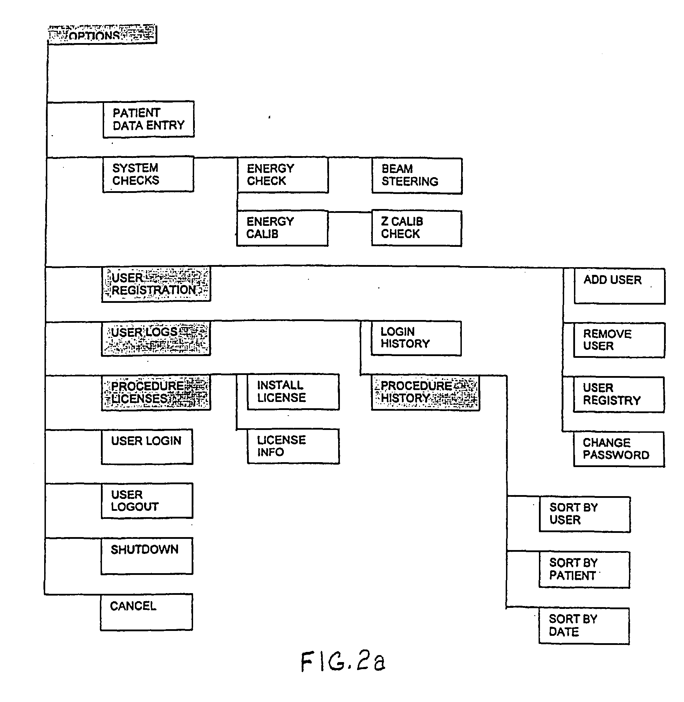

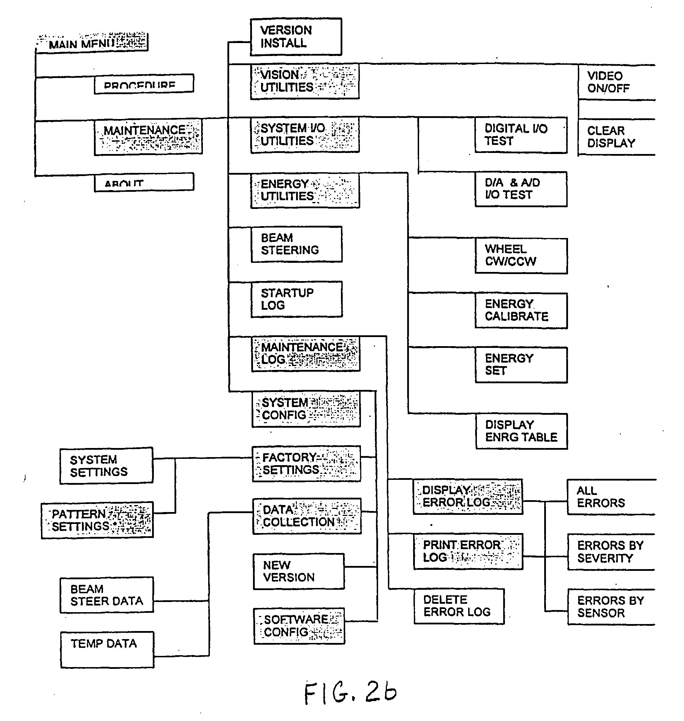

Referring now to Figure 2a-2b, navigational models are shown illustrating how a user interacts with the instrument software to access functionality and information. The navigational model is network designed to minimize the number of actions and the amount of time required of the user. Two networks are implemented, one for the user (inside the Procedure Window) and one for maintenance and R&D personnel (from the Main Menu).

### 1. Laser System Initialization

The instrument software utilizes a settings file for initialization and startup of the laser system. The settings file includes settings for the standard and maximum energy, Z scale, X, Y, and Z offset, beam steering X, Y origin, energy setting search steps, energy measurement conversion factor, and temperature measurement conversion factor.

### 2. Laser System Operator Interface

The laser system uses an operator interface to provide a flexible, easy-to-use environment for the operators to interact with the laser system. Preferably a graphical user interface (GUI) is used employing menus, buttons, and other graphical symbols that display various functions that may be performed to the operator. To execute most tasks, the operator points to an object and selects the object, for example by clicking on the object. A button or menu item followed by a menu mark (small triangle pointing down or to the right) indicates an available submenu from which additional items may be selected.

Various input points may be utilized or configured with the instrument software to enter or input data or information into the system. Some of these input devices are a keyboard, a trackball or mouse, a joystick, a variable focal length switch, and a footswitch.

### 3. System Security

The instrument software and use of the laser system is preferably protected through password protection. An encrypted, binary password file is used by the instrument software which includes the login name, password level, and an encrypted password string. In one embodiment, the system software prompts the user to enter a login name and password. Each user is assigned a passlevel. An "owner" user has the permission to add additional users with the same or lower passlevels, as well as remove users and change their password. Preferably, a history file is maintained by the instrument software to record the login/logout information of a user. Certain software and system functions are accessible where the user has the appropriate passlevel. The instrument software preferably has the following passlevels:

| **Passlevel** | **User Type** | **Description** |
|---|---|---|
| 0 | None | Login select only |
| 1 | User | Procedure Window only |
| 2 | Owner | Passlevel 0 and 1, plus password assignment and some maintenance privileges |
| 3 | Maintenance | Passlevel 0 and 1, plus access to the Maintenance Window |
| 4 | R&D personnel | Passlevel 0-3, plus access to R&D parameters, available in the Procedure Window |

A User Login Window is displayed every time system software is invoked or after a user logout of the system. Preferably, the User Login Window has a field for a user login name and a password field (using hidden characters).

Once the user has successfully logged into the system, the Procedure Window is displayed. Users with a passlevel 1 may not access any of the other windows. Users with a higher passlevel may close the Procedure Window and display the Base Window. The Base Window has menu option Procedure, Maintenance, About, and Help. Selecting the Procedure menu option displays the Procedure Window. The Procedure Window is used for normal procedure performing operations. The Maintenance menu option is displayed if the user has an authorized passlevel for maintenance. The Maintenance menu provides additional functions for maintenance personnel. Users may select an About menu option to display a window to convey information relating to company information and the current version of the instrument software. Additionally, the Base Window has a HELP menu item or button to provide access the system's help facility. Only Maintenance and R&D Personnel have read/write access (based on selected passlevel) to the system default and calibration files.

### 4. System Error Monitoring

The instrument software monitors all system errors during startup, idle state, and during the procedure and takes appropriate actions depending on the severity level of the error. Generally, interrupt '15' is triggered when a hardware detectable error is sensed. All errors are logged to an error log file. The error log file is a binary file which is used to capture the error time and date stamp, error description, sensor input number, severity, and system status (idle or running). Each of the errors may be assigned an error level. Depending on the error level, certain events occur. The following are the preferred error levels for an error:
- Level 1 - Notify user, procedure may continue
- Level 2 - Notify user, procedure may not start/continue
- Level 3 - Notify user, procedure may not start/continue, shut down laser
- Level 4 - Procedure may not start/continue, shut down laser & computer

Some of the error checks include checks for: AC Power Error, DC Power Error, Coolant Flow Switch Error, Oscillator Diode Temperature Out of Range, Oscillator Diode Off, Amplifier Diode Temperature Out of Range, Amplifier Diode Off, Galvanometer Error, Energy Error, Shutter 1 Feedback, Fire Feedback, FootSwitch, Coolant Level, Interlock Oscillator Mode Lock, Mode Lock Unstable, Shutter 2 Feedback, Variable Focal Lens Out.

For example, an error check for AC Power Error may be set at level 3. If the AC Power Error occurs during startup then startup will be terminated, the error is displayed and logged, and the laser turned off. If the AC Power Error occurs while the instrument is idle, then the error is displayed and logged, and the laser turned off. If the AC Power Error occurs during a procedure, then the procedure is terminated, the laser turned off, the error is displayed and logged.

A watchdog timer periodically send pulses to the laser control electronics to confirm that the instrument software is running. If the laser control electronics stop receiving these pulses, the entire system will shut down.

### 5. Procedure Window

The Procedure Window is available to an authorized user for performance of a procedure. The Procedure Window graphically displays, in real time, the procedure progress and provides various options to the user. Users with passlevel 3 (Maintenance) or higher may access the Systems I/O Utilities menu directly from the Procedure Window.

**Flap Patterns.** A Flap button is provided on the Procedure Window. Clicking this button disables all other Procedure Window buttons and opens a Predefined Flap Patterns Window. The user may perform an operation having flap characteristics by selecting from several predefined flap parameters. Patterns may be customized by changing parameter values inside a User Defined Flap Parameters Window. Parameter cross-validation is performed before accepting modified parameter. Two levels of access are provided for changing flap pattern parameters. Users with passlevel 1 (user) or 2 (owner) may change a limited set of parameters to create custom patterns. Users with passlevel 4 (R&D personnel) may change all parameters to create custom patterns.

The Predefined Flap Patterns Window displays a set of predefined selectable values for a number of flap parameters (highlighted values are defaults):
Upper Diameter (predefined values of ***8.0***, 8.5, 9.0 and 9.5 mm)
Depth in Cornea (predefined values of 150, ***160***, 170 and 180 um)
Hinge Angle (predefined value of ***60***)
Hinge Position (Nasal, Temporal or ***Superior****)*
Normal /Inverse Flap indicator (***Normal*** or Inverse)

The lower diameter is calculated based on the diameter and the side cut angle. The following Table 1 identifies the available Flap Pattern Parameters used by the instrument software:

**TABLE 1**

| **PARAMETER** | **DEFAULT** | **MIN** | **MAX** |
|---|---|---|---|
| Type (NORMAL/INVERSE) | NORMAL | - | - |
| Flap Thickness (µm) | 160 | 0 | 600 |
| Depth in contact glass (µm)/Normal only/ | 50 | 0 | 300 |
| Spiral 1,2 tangential spot separation (µm) | 12 | 1 | 30 |
| Spiral 1,2 radial spot separation (µm) | 10 | 1 | 30 |
| Spiral 1,2 energy (µJ) | 5 | 1 | 15 |
| Number of spirals | 1 | 1 | 10 |
| Spiral separation (µm) | 0 | 0 | 50 |
| Spiral depth (µm) /Inverse only/ | 100 | 0 | 600 |
| Horizontal overlap (µm) | 50 | 10 | 300 |
| Vertical overlap (µm) | 20 | 0 | 100 |
| Flap Diameter (mm) | 8.0 | 0.1 | 10 |
| Spiral 2 Diameter (mm) | 8.5 | 0.1 | 10 |
| Side cut energy (µJ) | 8.0 | 1 | 15 |
| Side cut angle (deg) | 60 | 30 | 120 |
| Side cut spot separation (µm) | 7 | 1 | 30 |
| Side cut layer separation (µm) | 5 | 1 | 50 |
| Hinge position (deg) /Normal only/ | 270 | 0 | 359 |
| Hinge angle (deg) /Normal only/ | 60 | 0 | 359 |
| Opening cut position (deg) | 0 | 0 | 359 |
| Opening cut angle (deg) | 0 | 0 | 359 |
| Opening cut separation (µm) | 0 | 0 | 50 |
| Number of opening cuts | 0 | 0 | 5 |
| Z radius (mm) | 0.0 | 0 | 50 |

The Predefined Flap Patterns Window displays the patient list, as entered previously in the Patient Data Entry Window. The following editable patient data fields are available (highlighted entries are mandatory):
***First name***, Middle Initial, ***Last Name***
***Date of Birth***
Patient ID
Description of Treatment: Spherical, Cylindrical, Axis
Corneal Thickness
Left or Right Eye
Previous Patient (if procedure was aborted)

The user is prompted with a warning message to double check the patient data entry. Info box and final shape projection are updated when changing predefined pattern parameters. A "NEXT" button allows the user to close the Predefined Flap Patterns Window. The user is then prompted to prepare the laser system for the procedure. A "TEST" button simulates the procedure without actually firing the laser (available to users with passlevel higher than 2). A "MORE" button when clicked opens the User Defined Flap Patterns Window. Selecting a "CANCEL" button closes Predefined Flap Patterns Window without proceeding with the procedure. The instrument software utilizes a binary, patient data entry file to include pre-procedure data for patients scheduled for a procedure. A binary, patient schedule file is used which includes the user login name, date of treatment, patient name, birth date, ID and treatment information.

In the User Defined Flap Patterns Window, the user may modify the following flap pattern parameters: Lower Diameter, Side Cut Angle, Hinge Position, Hinge Angle, Depth in Cornea, Spiral Energy and Side Cut Energy.

**Ring Patterns.** A Ring button is provided on the Procedure Window. Clicking this button disables all other Procedure Window buttons and opens a Predefined Ring Patterns Window. The user may perform an operation having ring characteristics by selection from several predefined ring parameters, discussed below. Patterns may be customized by changing parameter values inside a User Defined Ring Parameters Window. Two levels of access are provided for changing ring pattern parameters. Users with passlevel 1 (user) or 2 (owner) may change a limited set of parameters to create custom patterns. Users with passlevel 4 (R&D personnel) may change all parameters to create custom patterns.

The Predefined Ring Patterns Window displays a set of predefined selectable values for a number of flap parameters (highlighted values are defaults):
Inner Diameter (predefined values of 6.4, 6.6 and 6.8 mm)
Outer Diameter (predefined values of 8.6, 8.8 and 9.0 mm)
Cornea Thickness (editable from 500 to 750 □m, default 660 □m).
Incision Axis: Nasal, Temporal or **Superior**

The following Table 2 identifies the available Ring Pattern Parameters used by the instrument software:

**TABLE 2**

| **PARAMETER** | **DEFAULT** | **MIN** | **MAX** |
|---|---|---|---|
| Depth in Cornea (µm) | 440 | 0 | 600 |
| Outer Diameter (mm) | 8.8 | 4.0 | 10 |
| Inner Diameter (mm) | 6.6 | 4.0 | 9.9 |
| Starting Position (IN/OUT) | IN | - | - |
| Ring Tangential Spot Separation (µm) | 12 | 1 | 30 |
| Ring Radial Spot Separation (µm) | 10 | 1 | 30 |
| Ring Energy (µJ) | 7.0 | 1 | 15 |
| Number of Ring Layers | 1 | 1 | 10 |
| Ring Layer Separation (µm) | 10 | 1 | 30 |
| Entry Cut Energy (µJ) | 12 | 1 | 15 |
| Entry Cut Length (mm) | 1.2 | 0.8 | 1.5 |
| Entry Cut Thickness (µm) | 5 | 0 | 50 |
| Incision Axis (deg) | 270 | 0 | 359 |
| Entry Cut Spot Separation (µm) | 8 | 1 | 30 |
| Entry Cut Line Separation (µm) | 5 | 1 | 50 |
| Entry Cut Layer Separation (µm) | 5 | 1 | 50 |
| Depth in Contact Glass (□m) | 50 | 0 | 300 |
| Vertical Entry Cut Overlap (µm) | 20 | 0 | 100 |
| Z Radius (mm) | 0.0 | 0 | 50 |

The Predefined Ring Patterns Window displays the patient list, as entered previously in the Patient Data Entry Window. The following editable patient data fields are available (highlighted entries are mandatory):
***First name***, Middle Initial, ***Last Name***
***Date of Birth***
Patient ID
Description of Treatment: Spherical, Cylindrical, Axis
Corneal Thickness
Left or Right Eye
Previous Patient (if procedure was aborted)

The user is prompted with a warning message to double check the patient data entry. Info box and final shape projection are updated when changing predefined pattern parameters. A ''NEXT'' button allows the user to close the Predefined Ring Patterns Window. The user is then prompted to prepare the laser system for the procedure. A "TEST" button simulates procedure without actually firing the laser (available to users with passlevel higher than 2). A "MORE" button when clicked opens the User Defined Ring Patterns Window. Selecting a "CANCEL" button closes Predefined Ring Patterns Window without proceeding with the procedure.

In the User Defined Ring Patterns Window, the user may modify the following ring pattern parameters: Cornea Thickness, Depth in Contact Glass, Inner Diameter, Outer Diameter, Entry Cut Length, Entry Cut Thickness, and Entry Cut Position.

**Calibration.** The Procedure Window provides for selection of calibration of the laser. If the passlevel is lower than 3, the user may only perform a Spiral Alignment check for spiral pattern with limited parameters or an Energy Calibration check for Spot Pattern with limited parameters. Those users with a passlevel greater than 3 may perform the above calibration plus a Spatial Alignment for Spiral Pattern with additional parameters, Energy Calibration for Spot Pattern with additional parameters, and an X-Y Galvonometer Calibration for circle with programmable parameters. The following Table 3 identifies the Alignment Check (Spiral Parameters) used by the instrument software:

**TABLE 3**

| **PARAMETER** | **DEFAULT** | **MIN** | **MAX** |
|---|---|---|---|
| Energy (µJ) | 8.0 | 1 | 30 |
| Depth in Cornea (µm) | 160 | 0 | 1000 |
| Diameter(mm) | 9.0 | 0.1 | 10 |
| Starting Position (IN/OUT) | IN | - | - |
| Tangential Spot Separation (µm) | 15 | 1 | 30 |
| Radial Spot Separation (µm) | 15 | 1 | 30 |
| Spiral Layers | 1 | 1 | 10 |
| Layer Separation (µm) | 10 | 1 | 50 |
| Z Radius (mm) | 0.0 | 0 | 50 |

**Options Menu**. The Procedure Window provides, via an Options Menu, the selection of various options. In one embodiment these options are:
Patient Data Entry (for procedure pre-scheduling
System Checks
   Energy Check (to set energy level and measure energy with an energy meter)
   Energy Calibration
   Beam Steering
   Z Calibration Check (run a predefined circle pattern procedure, stop procedure twice, compute and display Z depht and tilt, if not within range, notify user and disable further procedures until calibration passes)
User Registration
User Logs
Procedures Licenses
User Login (selectable if logged out)
User Logout (selectable if logged in)
Shut down (user confirmation required followed by option selection: Shut Down Laser, Shut Down System and Cancel)
About menu item to show current software version
Cancel (closes the Procedure Window if passlevel is highter than 2)

**Options Menu -** *Patient Data Entry*. Patient data entry is entered by the user. Patient data is preferably stored in a patient data entry file. Multiple patient data entry (scheduling) is available to a user prior to treatment. The patient data entered by the user is stored in the user procedure file, as well as the patient data entry file. The instrument software provides the following editable patient data fields (highlighted entries are mandatory):
***First name***, Middle Initial, ***Last Name***
***Date of Birth***
Patient ID
Type of Treatment: Flap or Ring
Left or Right Eye
Diopter: Spherical, Cylindrical, Axis
Corneal Thickness

**Options Menu** - *User Registration.* A user with appropriate passlevel is able to add, remove, or change the password of another user with the same or lower passlevel. The instrument software uses a binary user registry file to include the user's first and last name, login name, password level, creator's login name and date of creation. Backup passwords and user registration files are created and updated via the instrument software. Corrupted or missing password and registry files may be restored. The user registration file may be printed and copied to external media, such as a floppy disk.

A user may be added or deleted and the password changed for the user. To add a new user, a user name is typed in along with a password (which is confirmed by entering the password again). A passlevel is selected for the user. Users may be deleted after confirming that the user actually should be deleted. Information about the user may be displayed, such as the user's name, login name, passlevel, the user who created the displayed login name and the creation date for the login name.

**Options Menu -** *User Logs.* A user is able to see his own Procedure history. A user with a passlevel 2 is able to view other user procedure history. The instrument software uses a binary login history file which includes the login names and login/logout times of all users. The login/logout history of a user may be viewed. A backup user log is automatically created by the instrument software. The login/logout history may be printed. The patient information and pattern parameter values used by the current user may be displayed, printed, and restored and back up made.

**Options Menu -** *Procure Licenses*. Procedure licenses may be loaded from a floppy disk and remaining procedure licenses viewed. The user is able to install procedure licenses for a particular laser system from a floppy disk. The user is able to view the number of procedure licenses for a particular laser system. The instrument software uses a binary user procedure file which includes the user login name, date and time stamp, patient name (optional), birth date (optional), ID (optional) and treatment information (optional), as well as the pattern calculation used by a particular user.

### 6. Maintenance Mode.

In the maintenance mode, an authorized user is allowed to access system hardware. Additionally, the maintenance mode of the instrument software provides certain utilities to assis testing, configuration and diagnostics of the laser system. It is important that the maintenance mode (low level hardware options) not be accessed by untrained and unqualified users. This mode allows direct access to the peripheral boards controlling the system. Various tests and functions may be performed in this mode.

**Digital I/O Test** - When the DIGITAL I/O UTILITIES option is selected, trained maintenance personnel can directly access all digital I/O's. Input states may be read or forced to ON/OFF state, outputs may be toggled ON/OFF.

**D/A & A/D I/O Test** - When this option is selected, trained maintenance personnel can directly set digital values controlling the analog voltage to the X, Y, Z galvanometers and the Energy Reference output, as well as read the values corresponding to the Laser Output Energy and Energy Reference Integrity analog signals.

**Energy Utilities** - The following energy utility options are available to authorized users:
Wheel to CCW Limit - Move energy wheel to CCW limit sensor.
Wheel to CW Limit - Move energy wheel to CW limit sensor.
Energy Wheel Calibration - When this option is selected, the energy wheel
calibration procedure is performed.
Energy Setting - The energy setting procedure consist of the following:
   - Select desired energy.
   - Use the Energy Wheel Position vs. Energy Value table to drive the energy wheel to the desired position.
   - Read back the actual energy value at this position with an energy meter.
   - If value does not match the selected energy value, move energy wheel left/right a predetermined number of steps until the value is found.
   - If the selected energy value is not found, look for the closest value, notify user of the actual value found.
Energy Table Display- Selecting this option displays the energy table values (energy vs. step) based on latest energy wheel calibration.

**Beam Steering Utilities** - Selecting this option performs the beam steering procedure and display computed **X** and **Y** origin values.

**Vision Utilities** - When this option is selected, the user may turn live video on/off.

**Error Log Utilities** - All run time instrument errors (alarm conditions) are logged and saved with a date and time stamp. The error is available for display, download, and printout. The error log may be deleted. A backup error log file is created and updated automatically. A corrupted or missing error log file may be restored. The error log file is viewable, printable, and deletable, as well as retrievable to a floppy disk. The following error log utility options are available to authorized users:
Display Error Log - Selecting this option displays all instrument run time errors (alarm conditions) sorted in a variety of manners.
All Instrument Errors - Selecting this option displays all run time errors in chronological order.
Errors Per Severity Category - Selecting this option displays all run time errors of a certain severity category in chronological order.
Errors Per Sensor - Selecting this option displays all run time errors caused by a user selectable sensor in chronological order.
Print Error Log - Selecting this option prints all instrument run time errors (alarm conditions) sorted in a variety of manners.
All Instrument Errors - Selecting this option prints out all instrument run time errors in chronological order.
Errors Per Severity Category - Selecting this option prints out all run time errors of a certain severity category in chronological order.
Errors Per Sensor - Selecting this option prints out all run time errors caused by a user selectable sensor in chronological order.
Delete Error Log - Selecting this option deletes the run time error log. Before deleting the error log file, a hard copy should be printed. The user is prompted for confirmation that the error log should be deleted.
Startup Log - All startup information, errors (alarm conditions) and energy wheel calibration results are logged and saved with a date and time stamp. Backup startup log file is created and updated automatically. A corrupted or missing startup log file may be restored. The startup log file is viewable, printable, and deletable, as well as retrievable to a floppy disk. The instrument software uses a binary, startup log file to include the time and date stamp, startup type, miscellaneous step status/values, measured maximum and minimum energy as well as any errors arid their severity.
Reset Errors - Selecting this option allows resetting any system error.

**System Settings Utilities-** The system settings options is available for users with passlevel 4 (R&D personnel) only. The Systems Settings function provides access to system settings: Z scale calibration factor, X,Y,Z offset, beam steering X and Y origin values and standard minimum and maximum energy values. A backup system settings file is created and updated automatically. The system settings file is restorable. The system settings is editable, viewable, and printable, as well as retrievable to a floppy disk.

**Pattern Settings Utilities -** The pattern settings options is available for users with passlevel 4 (R&D personnel) only. Factory preset pattern parameters are provided for both flap and ring patterns. A backup file is created and updated automatically. A corrupted or missing pattern settings file may be restored. The pattern settings is editable, viewable, and printable, as well as retrievable to a floppy disk. The following pattern settings may be modified:
Flap Factory Settings - The Flap Factory Settings are modifiable. Parameter cross-validation is performed before accepting the modified parameter values.
Ring (Tunnel) Factory Settings - The Ring (Tunnel) Factory Settings are modifiable.
Parameter cross-validation is performed before accepting the modified parameter values.
Spatial Calibration Settings - The Spatial Calibration Settings are modifiable. These settings control the spiral pattern properties.
Energy Calibration Settings - The Energy Calibration Settings are modifiable. These settings control the spot pattern properties.
Galvo Gain Settings - The Galvo Gain Settings are modifiable. These settings control the circle pattern properties.
Calibration Check Settings - The Z Calibration Check Settings are modifiable. These settings control the circle pattern properties used in Z Calibration Check.

**Data Collection Utilities** - The data collection options are available for users with passlevel 3 (maintenance) or higher. Beam steering quadrature detector measurements are recorded before and after the execution of a beam steering procedure. The beam steering quadrature detectors analog inputs from 4 channels A, B, C and D are recorded. The voltage from each channel is recorded.

Temperature readings are periodically recorded to a data file. Voltage from thermistors is converted to obtain temperatures. The temperature readings are preferably taken every thirty minutes. The data collection options allow users to access the data collection files.

Beam Steering Data - This option provides access to the Beam Steering Data File. A backup beam steering data file is created and updated automatically. A corrupted or missing beam steering data file may be restored. The beam steering data file is viewable and, printable, as well as retrievable to a floppy disk.

Temperature Data - This option provides access to the Temperature Data File. A backup temperature data file is created and updated automatically. A corrupted or missing temperature data file may be restored. The temperature data file is viewable, printable, as well as retrievable to a floppy disk.

**Other Utilities** - Other utility options are available. Some of these include:
Set Location Time - This option allows setting and saving the local time.
Install New Version - This option allows the installation from floppy disk(s) of a new software version.
Software Configuration - This option allows the enabling or disabling of certain factory installed options.

Beam Centering - A vision machine is used to center the laser beam to the desired location in the pupil before performing a procedure. The concentricity of the laser beam and pupil is preferably 0.1 mm or better. The beam centering may be disabled by the user.

Calibration: A vision calibration procedure returns the galvo output / pixel values (X and Y) needed to send the offset value to the galvanometers. The following operations take place during calibration:
- Calibration Start command is sent by the Instrument Controller to the *vision* process.
- The *vision* process performs the calibration and returns the status flag and calibration factors (X & Y).
- If calibration failed, the beam alignment cannot be executed, reset X, Y offset values to 0, notify user, procedure may be performed.

Alignment: The vision alignment return the offset value in pixel (X and Y) needed to center the laser beam to the desired location in the pupil. The following operations take place during alignment:
- Align Start command is sent by the Instrument Controller to the *vision* process.
- The *vision* process performs the calibration and sends to the Instrument Controller the status flag and offset values (X & Y).
- If alignment fails, the operator is notified, reset X, Y offset values to 0, procedure may continue.

### 7. Pattern Defining Procedure

The instrument software of the laser system generates the X, Y, Z values used in positioning the X, Y and Z galvanometers for focusing the laser beam for photodisruption. The pattern calculation may be performed based on a set of factory preset parameters or by using custom parameters modified by the user. Additionally, authorized users with appropriate passlevels may modify additional pattern parameters to provide a wider range of settings.

The process of creating a flap can be divided into two general segments. A spiral path of photodispruption creates is the creation of a horizontal circular resection inside the cornea. The side cut is a vertical resection around the perimeter of the horizontal resection. The order of the segments depends on the type of the Flap Pattern. Basically two different types are defined. Normal Flap Pattern, where the spiral path of photodispruption is the first segment and it is followed by the side cut. Inverse Flap Pattern, where the side cut is the first segment and it is followed by the spiral path of photodisruption on the top of the side cut.

The spiral process starts when the laser system sets the mirror X-Y galvanometers to the center of the laser's visual field. The Z-galvanometer is then set to a position that focuses the laser's light to the desired depth (typically 160 µm) below an aplanation contact lens. When the command to fire the laser is given, the safety shutter is opened, allowing light to enter the delivery system. At the same time, the X-Y galvanometers are actuated to move the focus in a circular path. Beginning at the visual center and proceeding in a circular path about the visual center, the laser focuses a sequence of pulses in a horizontal plane. The focus typically traverses the circle in a counter-clockwise fashion when viewed from the user's perspective. The speed with which the laser focus traverses the circle depends on the desired spot separation between focused pulses of light. The radius of each successive circle is incrementally increased by a fixed amount until the maximum radius of the horizontal resection is reached. When the final circle is completed, the laser shutter is closed. In this manner, a horizontal circular resection is defined at the desired depth within the cornea.

The side cut, or the vertical resection, begins when the instrument sets the X-Y galvanometers to a position just inside of (typically 50 µm) the circumference of the horizontal resection, nominally at the nine o'clock position. The Z- galvanometer is then set to a position that focuses light to a depth slightly below (typically 20 µm) the plane of the horizontal resection. The laser shutter is then opened and the X-Y galvanometers actuated to move the focus in a circular path. The speed with which the laser focus traverses the circle depends on the desired spot separation between focused pulses of light. Typically, the focus traverses the circle in a counter-clock wise fashion when viewed from the user's perspective. In this manner, the laser focuses a sequence of pulses into a circle that inscribes the horizontal resection. When the circle is complete, the Z-galvanometer is repositioned to a depth slightly above the circle. This process is repeated until the final circle is created a few microns into the aplanation contact lens. Simultaneously with the creation of vertically displaced circles, the radius of each successive circle is incrementally increased by a fixed amount until the maximum radius of the vertical resection is reached.

The process outlined above defines the general rule for creating a vertical resection. There are two exceptions to this rule; the first is creation of a flap hinge. The hinge is that part of a circle where no photodisruption is allowed. The hinge is an arc defined by its position and angle. When the laser focus reaches a pre-determined position along its circular path, the X-Y galvanometers stop and reverse their coordinated motion with the next circle above the previous circle. This is done for each circle comprising the vertical resection. In this manner, a portion of the vertical resection is masked for the creation of a flap hinge.

The second exception to the vertical resection rule is the creation of an opening cut. For the purpose of this discussion, counterclockwise motion is termed "forward" and clockwise motion is termed "backward." At a predetermined position along the resection's circular path, the X-Y galvanometers stop and reverse their coordinated motion without altering the depth of the cut. In effect the focus spot reverse its direction of motion and retrace its path along the circle. While traversing backward and at a new position, the X-Y galvanometers reverse their coordinated motion for a second time. The laser focus resume its forward motion and continue until the circle is complete. This process is repeated for each circle comprising the vertical resection. The arc length over which the focus retraces itself is termed the "opening cut."

### 8. Ring (Tunnel) Procedure

The process of creating a ring (tunnel) can be divided into two general segments. The first phase is the creation of a horizontal circular resection inside the cornea. Its shape is that of a planar ring. The ring resection is followed by an entry cut resection at a certain position on the surface of the horizontal resection.

The ring creation of a ring pattern starts when the instrument sets the mirror X-Y galvanometers to the start position of the first circle. The first circle may be the smallest inner or the biggest outer circle. Normally the process is started from the inner circle. The Z-galvanometer is then set to a position that focuses the laser's light to a desired depth (typically 440 µm) below the aplanation contact lens. When the command to fire the laser is given, the safety shutter is opened, allowing light to enter the delivery system. At the same time, the X-Y galvanometers are actuated to move the focus in a circular path. Beginning at the inner circle and proceeding in a circular path about the visual center, the laser focuses a sequence of pulses in a horizontal plane. The speed with which the laser focus traverses the circle depends on the desired spot separation between focused pulses of light. The focus typically traverses the circle in a counter-clock wise fashion when viewed from the user's perspective. The radius of each successive circle is incrementally increased by a fixed amount until the maximum radius of the ring cut is reached. When the final circle is completed, the laser shutter is closed. In this manner, a horizontal circular resection is created at the desired depth within the cornea.

The entry cut resection is a vertical plane from the ring level up to the contact lens. The cut begins when the instrument sets the X-Y galvanometers to a predefined position on the circumference of the horizontal resection, nominally at the twelve o'clock position. The Z-galvanometer is then set to a position that focuses light to a depth slightly below (typically 20 µm) the plane of the horizontal resection. The laser shutter is then opened and the X-Y galvanometers actuated to move the focus along a straight line path. In this manner, the laser focuses a sequence of pulses into a line that inscribes the horizontal resection. When a line is complete, the Z-galvanometer is repositioned to a depth slightly above the line. The instrument then executes a second line at the same length directly above the first line. This process is repeated until the final line is created a few microns into the aplanation contact lens.

### Instrument Software - Technical Architecture

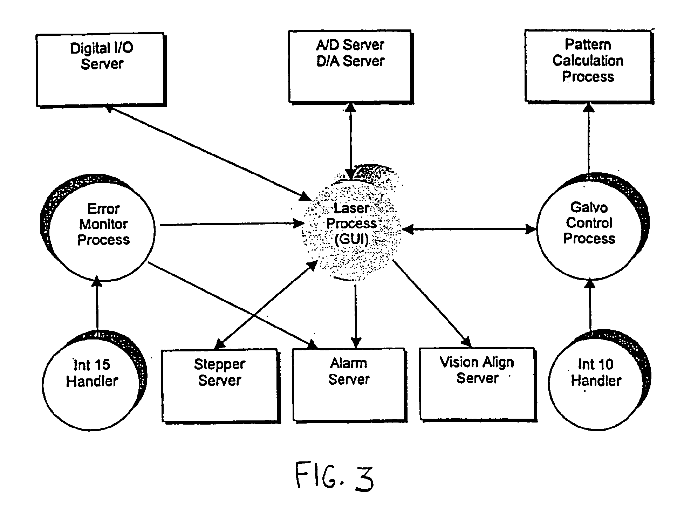

Figure 3 is an instrument process context diagram. The process context diagram describes the major processes that are performed by the instrument software. The process context diagram outlines the instrument software design from a function point of view. Each function shown is shown at a high level. In the diagram, servers are represented by rectangles, and other processes by circles.

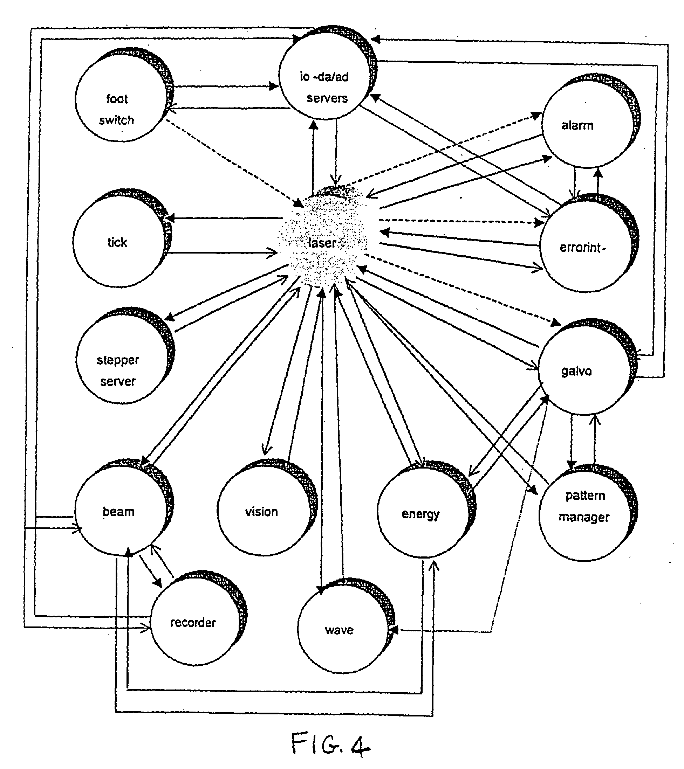

Figure 4 is a top level process communication hierarchy diagram. Main inter-process communications are shown using arrows to indicate the sender and the receiver of the communications. For example, an arrow from process A to process B indicates that process A sends a message to B and B receives the message. from A. In all cases, the process that sends the message is reply blocked until a reply is returned. Messages are represented by solid lines, proxies by dashed lines and queue messages by dotted lines. The main inter-process communications are set forth below.

***laser* process.** The *laser* process controls and coordinates the instrument actions and provides an operator interface. The laser process spawns all other processes in the system. Upon invocation of the instrument software, the *laser* process is spawned by a shell. The main function of the *laser* process is to control and coordinate the actions of the instrument. During the initialization of the *laser* process the following action are taken:
- Begin a new QNX session, make the *laser* process a session leader.
- Open queues.
- Spawn child processes.
- Receive the proxy ids for the processes *alarm, galvo, errorint_test*, and *errorint*.
- Set a new tick size (1 msec) for the operating system scheduler.
- Load configuration and factory default data files.
- Set up input procedures for receiving non-Photon messages from other processes (*beam*).
- Display user login dialog, wait for login, validate password, establish passlevel.
- Once logged in, the user may proceed with the instrument startup procedure or open the Patient Data Entry Window.
- Perform instrument startup procedure.
- If startup is successful, set up input procedures for receiving non-Photon messages from other processes (*errorint*, *energy*, *vision*).

***ioserver* process.** Upon invocation of the instrument software, the *ioserver* process is spawned by the *laser* process. The main function of the *ioserver* process is to accept digital input/output commands. During the initialization of this process the following actions are taken:
- attach a name so other processes can locate *ioserver's* process identification
- locate '*estop*' process
- initialize digital I/O board
- initialize digital I/O's on multifunction I/O board
- create a 2 msec timer proxy attached to itself

During normal operations, the sequence of actions performed by the *ioserver* process are as follows:
- wait for a request or timer proxy
- if IO_TYPE request received, turn output on/off (if any), set up service to wait for an input state (if any requested)
- if IOTEST_TYPE request received, perform client request, reply to client

***daserver* process.** The *daserver* process reads analog to digital inputs and writes digital to analog outputs, as well as preloads the counter used as the laser's repetition rate generator. Upon invocation of the instrument software, the daserver process is spawned by the laser process. The *daserver* process sends back results based on the state of the A/D converters. During the initialization of this process, the follow actions are taken:
- attach a name so other processes can locate *daserver's* process identification
- locate '*estop*' process
- initialize A/D-D/A I/O board

During normal operations, the sequence of actions performed by the *daserver* process are as follows:
- wait for a command from a client
- process command request
- if command is a read analog input, access directly hardware, read input value and return to client
- if command is a write analog output, access directly hardware and write output value
- if command is set repetition rate, change frequency generator counter divider

***alarm* process**. The *alarm* process gets notified of an error condition during the procedure or while the instrument is idle and logs all errors to a file. Upon invocation of the instrument software, the *alarm* process is spawned by the laser process. The main function of the *alarm* process is to save to the file the alarms as they occur (during the idle state of the instrument) and store them in memory and save them to disk after the procedure is complete (during the procedure). During the initialization of the *alarm* process the following actions are taken:
- Attach a name so other processes can locate *alarm's* process identification.
- Attach a proxy to itself and make its ID available to the *laser* processes (its parent). Receiving this proxy from the *laser* process is indication that the procedure is in progress and all errors should be stored temporarily in memory.

During normal operations, the sequence of actions performed by the *alarm* process are as follows:
- Wait for an error message or proxy.
- If proxy received, set the 'running' flag, meaning that all messages are stored in memory and no disk I/O are performed until the procedure is over.
- If a true error message is received, the message is saved to file right away (if system is idle) or, if the procedure is in progress, are stored to memory until the procedure is done and then saved to file.
- A special error message code "0000" is used to indicate to the *alarm* process that the procedure is complete and all errors stored in memory may be saved to file and the 'running' flag may be reset.
- Saving to file consists of opening the error log file, writing a record for each error and closing the error log file (all dynamic memory allocated are freed at that time).
- The error log file structure is as follows: date and time stamp, error code to indicate input number and severity, error message text and the running/idle flag
- A backup log file is generated.

***galvo* process.** The *galvo* process initiates a pattern point computation every time an interrupt is generated by the hardware and controls the positioning of the X, Y, and Z galvanometers, as well as opening and closing the shutter. Upon invocation of the instrument software, the *galvo* process is spawned by the *laser* process. During the initialization of this process, the follow actions are taken:
- Attach a name so other processes can locate *galvo's* process identification.
- Locate '*ioserver*,' '*ptrn_mngr*,' '*galvo_home*' and '*wave*' processes identification.
- Open int10_test and energy queues.
- Attach a proxy to itself and make its ID available to the *laser* process (its parent). Receiving this proxy from the *laser* is an indication that the *laser* process needs to initiate a communication session with the *galvo* process.
- Attach an interrupt '10' to itself and make it generate a proxy every time the interrupt is generated.
- Attach to itself a 1 ms timer proxy (used in demo mode only).
- Check hardware interrupt '10', after one hit write message into int10_test queue.

During normal operations, the sequence of actions performed by the *galvo* process are as follows:
- Wait for an interrupt proxy (or timer proxy in demo mode), laser communication proxy or a message from the *energy* process.
- If laser communication proxy is received, initiate a communication session with the *laser* process by sending it an empty message. The reply message from the *laser* process indicates the service type to be performed by the *galvo* process: pattern type selection, start procedure, reset procedure, initialize new pattern, dummy (used internally), restart.
- If an interrupt proxy is received and the procedure is on, request new X, Y, Z values from the pattern manager process (flap, ring or test pattern). The received values are output to the galvo D/A converters and also send to the laser process for display. The stop status are embedded in the reply message from the *laser* process. Special flags are used as end of pattern (shutter closes, procedure ends) and end of section markers (shutter closes and energy are set, shutter reopens). During the procedure a flag is set and ever 750 msec a watchdog timer trigger is toggled to update the hardware that no critical system lockups exist. While not performing a procedure, the *galvo* process sends a proxy to the *wave* process.
- If a message from the *energy* process is received, it means that energy setting for next section is complete, the shutter is reopened and the procedure resumes.

***energy* process.** The *energy* process performs the energy setting function. Upon receiving a command, this process positions the energy wheel to a previously calibrated position corresponding to the desired energy. During the initialization of this process the following actions are performed:
- Open the energy queue.
- Locate '*ioserver*,' and *'daserver'* processes identification.
- Remain blocked on queue until a command is written to it.

During normal operations, the sequence of actions performed by the *energy* process are as follows:
- Wait for a command to be written into the queue to start the energy setting process. Commands may be written by the *laser, beam* or *galvo* process.
- Two types of commands are valid: reload energy table from file and set energy.
- If a reload energy table command is received (after a new energy wheel calibration), the new values are loaded from file into memory for future use.
- If a set energy command is received, the energy wheel is turned to the position corresponding to the desired value, then a best-match search is performed within a given range in both directions around the position. A completion message is sent to the process that initiated the energy setting request.

***errorint* process.** The *errorint* process handles the interrupt '15' generated by hardware in case of a system error. The process identifies the cause of error, take the necessary steps to protect the hardware and notifies other processes that an error has occurred. Upon invocation of the instrument software, the *errorint* process is spawned by the *laser* process. When a proxy is received, the process identifies the cause of the error and notify the *laser* and *alarm* processes. During the initialization of the process the following actions are performed:
- Attach a proxy to itself and make its identification available to the *laser* process (its parent). Receiving this proxy from the *laser* process is an indication that the startup has been successfully completed.
- Locate *ioserver* and *estop* processes.
- Attach a GUI proxy to itself to be triggered by the interrupt handler.
- Attach a handler to hardware interrupt '15', the handler triggers the proxy above.
- Locate *alarm* process.

During normal operations, the sequence of actions performed by the *errorint* process are as follows:
- Wait for an interrupt '15', trigger proxy.
- When proxy is received, disable further interrupts, identify the error cause and its severity.
- Based upon the severity level and type of error, take protective action by turning off critical outputs.
- Notify the *laser* and *alarm* processes that an error has occurred.
- If a GUI proxy is received, re-enable error interrupt '15'.

***errorint-test* process.** The *errorint-test* process is responsible for checking during startup that the error interrupt '15' is operational. Upon invocation of the instrument software, the *errorint_test* process is spawned by the *laser* process.

During the initialization of this process the following actions are performed:
- Attach itself a proxy and make it available to the *laser* process.
- Open the error interrupt test queue.
- Locate the *ioserver* process.

**[00109]** During normal operations, the sequence of actions to be implemented by the *errorint-test* process is performed as follows:
- Wait for proxy from *laser* process (startup in progress).
- When proxy is received, attached handler to hardware interrupt '15' so that when an interrupt is received, a proxy is generated.
- Turn on INT15_TEST output to trigger hardware interrupt '15'.
- When interrupt '15' is received, write data to error interrupt test queue, and unattach interrupt '15' handler.

***estop* process.** The *estop* process is responsible for shutting down power to all critical devices in the system in case of an internal software failure. Upon invocation of the instrument software, the *estop* process is spawned by the *laser* process. During the initialization of this process, the following actions are taken:
- Attach a name so other processes can locate *estop's* process identification.

During normal operations, the sequence of actions performed by the *errorint* process are as follows:
- Wait for a message.
- If the request is of "STOP" type, then shut down laser power, and send message to the *laser* process.

***footswitch* process.** The *footswitch* process is responsible for monitoring the footswitch instrument of the instrument. Upon invocation of the instrument software, the *footswitch* process is spawned by the *laser* process. During the initialization of this process the following actions are performed:
- Locate *ioserver* process.
- Create a start proxy attached to the *laser* process.
- Create a stop proxy attached to the *laser* process.
- Check current state of footswitch (open or close).

During normal operations, the sequence of actions performed by the *footswitch* process are as follows:
- If footswitch is open, test for footswitch closed.
- If a change of state is sensed, trigger start proxy to laser.
- If foot switch is closed, test for footswitch open.
- If a change of state is sensed, trigger stop proxy to laser.

***beam* process.** The beam process is responsible for performing the *beam* steering procedure. Upon invocation of the instrument software, the *beam* process is spawned by the *laser* process. During the initialization of this process the following actions are performed:
- Open beam and energy queues.
- Locate *ioserver* process.
- Locate *daserver* process.
- Locate *recorder* process.

During normal operations, the sequence of actions performed by the *beam* process are as follows:
- Wait for a command from the *laser* process to be written into the queue to start the beam steering process. The beam steering process includes:
   a) Write set energy command to the energy queue, wait for completion message from energy, reply
   b) Close beam steering shutter, check shutter closed.
   c) Open main shutter, check shutter open.
   d) Read the analog inputs corresponding to the 4 quad detectors, send values to the *recorder* process for saving to file.
   e) Read the analog inputs corresponding to the 4 quad detectors server times, average readings.
   f) Compute normalized value.
   g) Compute x origin.
   h) Compute y origin.
   i) If the computed x and/or y origins are not within tolerance from the factory preset values, send direction and pulse command (burst of pulses) to the respective axes.
   j) Repeat from step e) several times or until x and y origins within tolerance.
   k) Read the analog inputs corresponding to the 4 quad detectors, send values to the *recorder* process for saving to a file.
   l) Close main shutter, check shutter closed.
   m) Open beam steering shutter, check shutter open.
   n) Send to *laser* message with PASS/FAIL status and computed x, y origin values.

***recorder* process**. The *recorder* process is responsible for saving to file the beam steering quadrature detector values (before and after beam steering procedure), as well as periodically reading and saving to file the temperature sensor values. Upon invocation of the instrument software, the *recorder* process is spawned by the *laser* process. During initialization of this process the following actions are performed:
- Attach itself a name.
- Locate *daserver, estop* processes.
- Start a thirty minute proxy timer.

During normal operations, the sequence of actions performed by the *recorder* process are as follows:
- If proxy is received, read the analog inputs connected to the three temperature sensors, and if the procedure in progress flag is reset, save values with date and time stamp to file. The file structure is a date/time, temp sensor, temp sensor, temp sensor.
- If message is received from *laser* process, then set/reset procedure in progress flag.
- If message is received from *beam* process, save quadrature detector values with date/time stamp and pre/post beam steering procedure flag to file The file structure is a date/time, pre/post beam steering flag, quad detector value 1, quad detector value 2, quad detector value 3, and quad detector value 4.

***wave* process.** The wave process is responsible for outputting to the X, Y, Z galvos values corresponding to the sine, square, or circle test shapes. Upon invocation of the instrument software, the *wave* process is spawned by the *laser* process. During the initialization of this process the following actions are performed:
- Attach itself a name.
- Locate *daserver, estop* processes.
- Receive block and wait for messages.

During normal operations, the sequence of actions performed by the *wave* process are as follows:
- If proxy is received from the *galvo* process and the wave generator is on, output to the X, Y, Z galvos analog value corresponding to the next computed point of current wave shape.
- If message is received from *laser* process, turn wave generator on/off, select shape and channel and update the frequency, amplitude and offset of the pattern.
- If start repetition rate message is received from *laser* process, start counting proxies, reply with empty message.
- If stop repetition rate message is received from *laser* process, stop counting proxies, reply with total number of proxies.
- Locate *daserver, estop* processes.
   Receive block and wait for messages.

***vision* process.** *The vision* process is responsible for turning on/off the live video image. Upon invocation of the instrument software, the *vision* process is spawned by the *laser* process. During the initialize process the following actions are performed:
- Locate *estop* process.
- Open vision queue.
- Setup video board for RGB camera video input processing.

During normal operations, the sequence of actions performed by the *vision* process are as follows:
- Wait for a command from the *laser* process to be written into the queue to start the vision process.
- When command is written, perform one of the following functions: live video on, live video off.
- Setup video board for RGB camera video input processing.

***pattern manager* process.** The *pattern manager* process is responsible for handling all the tasks relating to pattern computation. It includes the validation of the pattern parameters and the calculation of the X, Y, Z coordinates controlling the laser beam path. Upon invocation of the instrument software, the *pattern manager* process is spawned by the *laser* process. The main function of the *pattern manager* process is the implementation of the following predefined patterns:
Clinical patterns
   - Flap (Normal and Inverse) Pattern
   - Ring (Tunnel) Pattern
Test patterns:
   - Spiral Pattern for Spatial Alignment
   - Circle Pattern for Galvanometer Calibration
   - Spot Pattern for Energy Calibration

The *pattern manager process* receives messages from the *laser* and *galvo* processes. The *laser* process initiates a parameter validation after each parameter change on the GUI. When the user selects a pattern and hits the fire button the on the GUI, the *galvo* process asks the *pattern manager* process to initialize the calculation process of the selected pattern. When the pattern execution is started the *galvo* process (upon receiving an interrupt) requests the *pattern manager* process to calculate the next XYZ coordinate until the end of pattern. Once a pattern is finished the pattern manager remains receive blocked until the next parameter validation or pattern execution.

During the initialization of this process the following action are performed:
- Attach a name so other processes can locate *pattern manager*'s process
- Locate the *estop* process.

During normal operations, the sequence of actions to be implemented by the *pattern manager* process are performed as follows:
- Wait for a message from another process (*laser* or *galvo*).
- When a message is received, the *pattern manager* process identifies the requested service, which may be one of the following:
   Parameter validation: This is a parameter cross-validation, the values of the different parameters have to be checked relative to each other to make sure every point of the pattern remains within the optical zone of the system. If the validation fails, the *pattern manager* process returns an error code to the *laser* process.
   Pattern initialization: The initialization of a pattern execution should be done right after clicking the Fire button. The initialization includes first an additional parameter validation, as it is useful to check the default pattern parameter values even if not changed by the user. If the initialization is completed successfully the *pattern manager* process returns the estimated procedure time and the first set of XYZ points to the *galvo* process.
   Vision offset check: The rechecking of parameters is necessary if the vision machine calculated a new set of XY offset values. This may occur after pattern initialization and before XYZ calculation only.
   XYZ coordinate calculation: This service can be requested after pattern initialization only. The *pattern manager* process calculates new XYZ coordinate values based on the initialized pattern and its parameters. The new XYZ values are used to move the laser beam to the next position. A pattern may consist of different geometrical shapes called segments. Based on the parameters the different segments can be executed by different laser beam energy level. The *pattern manager* process recognizes the last point of the current segment and it returns the new energy values of the next segment to the *galvo* process. If the calculation reached the last point of the pattern the process returns a pattern end status.
- Based on the requested service and the selected pattern the *pattern manager* process performs this service and returns the appropriate status code.
- Once a pattern execution is finished the pattern manager is ready to receive other parameter validation or pattern initialization service requests.

The *galvo-home* process is responsible for homing the galvos on power up and after the completion of a procedure, as well as moving the galvos slowly to a pre-selected position. Upon invocation of the instrument software, the *galvo-home* process is spawned by the laser process. During the initialization of this process the following actions are performed:
- Attach name to itself
- Open queue.
- Locate the daserver process.

During normal operations, the sequence of actions to be implemented by the *galvo-home* process is performed as follows:
- Wait for a write to queue from the laser or *galvo-home* process.
- When valid data is written to queue, send to *laser* process, perform moving X,Y,Z galvo axes slowly to commanded location. Home for X,Y,Z axes is 0V.

**[00128]** Although the present invention and its advantages have been described in detail, it should be understood that various changes, substitutions and alterations can be made herein without departing from the spirit and scope of the invention as defined by the appended claims. Moreover, the scope of the present application is not intended to be limited to the particular embodiments of the process, machine, manufacture, composition of matter, means, methods and steps described in the specification. As one of ordinary skill in the art will readily appreciate from the disclosure of the present invention, processes, machines, manufacture, compositions of matter, means, methods, or steps, presently existing or later to be developed that perform substantially the same function or achieve substantially the same result as the corresponding embodiments described herein may be utilized according to the present invention. Accordingly, the appended claims are intended to include within their scope such processes, machines, manufacture, compositions of matter, means, methods, or steps.

## Claims

1. A method for control of a laser system for photodisruption of a target, said method comprising the steps of:
directing a laser beam of said laser system to perform a horizontal resection procedure based upon predetermined pattern parameters;
displaying an image of the target within a display device;
wherein said predetermined pattern parameters include a variable Z-radius setting.

2. The method of claim 1, further comprising directing the laser beam to perform a vertical resection procedure.

3. The method of one of claims 1 to 2 whereby said horizontal resection is created with a Z-radius ranging from greater than 0 mm to 50 mm.

4. The method of one of claims 1 to 3, wherein the horizontal resection is formed by directing said laser beam to photodisrupt a portion of said target and proceed in a circular path about a predetermined point of said target, and continuing directing said laser beam to photodisrupt material of said target in concentric circles to create a desired horizontal resection diameter.

5. The method of one of claims 1 to 4, wherein the horizontal resection is formed by directing said laser beam to photodisrupt a portion of said target, and proceed in a spiral path about a predetermined point of said target to create a desired horizontal resection diameter.

6. The method of one of claims 1 to 5, wherein the pattern parameters include one or more of the following parameters: flap diameter, flap thickness, depth in target; depth in contact glass, hinge angle, hinge position, spiral tangential spot separation, spiral radial spot separation, spiral energy, spiral separation, spiral depth, spiral diameter, number of spirals, horizontal overlap, vertical overlap, side cut energy, side cut angle, side cut spot separation, side cut layer separation, opening cut position, opening cut angle, opening cut separation, number of opening cuts, Z radius, and normal/inverse flap indicator.

7. The method of claim 6, wherein the flap parameter for flap thickness ranges from 0 (µm) to 600 (µm).

8. The method of one of claims 5 to 7, wherein the flap pattern parameter for depth in contact glass ranges from 0 (µm) to 300 (µm).

9. The method of one of claims 5 to 8, wherein the flap pattern parameter for spiral tangential spot separation ranges from 1 (µm) to 30 (µm).

10. The method of one of claims 5 to 9, wherein the flap pattern parameter for spiral radial spot separation ranges from 1 (µm) to 30 (µm).

11. The method of one of claims 5 to 10, wherein the flap pattern parameter for spiral energy ranges from 1 (µJ) to 15 (µJ).

12. The method of one of claims 5 to 11, wherein the flap pattern parameter for the number of spirals ranges from 1 to 10.

13. The method of one of claims 5 to 12, wherein the flap pattern parameter for the spiral separation ranges from 0 (µm) to 50 (µm).

14. The method of one of claims 5 to 13, wherein the flap pattern parameter for the spiral depth ranges from 0 (µm) to 600 (µm).

15. The method of one of claims 5 to 14, wherein the flap pattern parameter for the horizontal overlap ranges from 10 (µm) to 300 (µm).

16. The method of one of claims 5 to 15, wherein the flap pattern parameter for the vertical overlap ranges from 0 (µm) to 100 (µm).

17. The method of one of claims 5 to 16, wherein the flap pattern parameter for the flap diameter ranges from 0.1 (mm) to 10 (mm).

18. The method of one of claims 5 to 17, wherein the flap pattern parameter for the spiral diameter ranges from 0.1 (mm) to 10 (mm).

19. The method of one of claims 5 to 18, wherein the flap pattern parameter for the side cut energy ranges from 1 (µJ) to 15 (µJ).

20. The method of one of claims 5 to 19, wherein the flap pattern parameter for the side cut angle ranges from 30 (deg) to 120 (deg).

21. The method of one of claims 5 to 20, wherein the flap pattern parameter for the side cut spot separation ranges from 1 (µm) to 30 (µm).

22. The method of one of claims 5 to 21, wherein the flap pattern parameter for the side cut layer separation ranges from 1 (µm) to 50 (µm).

23. The method of one of claims 5 to 22, wherein the flap pattern parameter for the hinge position ranges from 0 (deg) to 359 (deg).

24. The method of one of claims 5 to 23, wherein the flap pattern parameter for the hinge angle ranges from 0 (deg) to 359 (deg).

25. The method of one of claims 5 to 24, wherein the flap pattern parameter for the opening cut position ranges from 0 (deg) to 359 (deg).

26. The method of one of claims 5 to 25, wherein the flap pattern parameter for the opening cut angle ranges from 0 (deg) to 359 (deg).

27. The method of one of claims 5 to 26, wherein the flap pattern parameter for the opening cut separation ranges from 0 (µm) to 50 (µm).

28. The method of one of claims 5 to 27, wherein the flap pattern parameter for the number of opening cuts ranges from 0 to 5.

29. The method of one of claims 5 to 28, wherein the flap pattern parameter for the Z-radius ranges from 0 (mm) to 50 (mm).

30. The method of one of claims 1 to 29, further comprising directing said laser beam to photodisrupt a portion of said target about the circumference of said horizontal resection to create a vertical resection.

31. The method of claim 30, further comprising directing the laser beam to begin photodisruption of said target at a position just inside of the circumference of the horizontal resection.

32. The method of claim 30, further comprising directing the laser beam to begin photodisruption of said target at a position to a depth slightly below the plane of the horizontal resection.

33. The method of claim 30, wherein a portion of the circumference for said desired horizontal resection is not photodisrupted, thereby forming a hinge for said desired horizontal resection.

34. The method of claim 30, wherein the vertical resection may be at an angle other than 90 degrees relative to the plane of the horizontal resection.

35. The method of one of claims 1 to 34, wherein the pattern parameters include ring pattern parameters for the creation of a ring-shaped resection of said target.

36. The method of claim 35, wherein the ring pattern parameters include one or more of the following parameters: inner diameter, outer diameter, target thickness, and incision axis.

37. The method of one of claims 35 to 36, wherein the ring pattern parameter for the depth in the target ranges from 0 (µm) to 600 (µm).

38. The method of one of claims 35 to 37, wherein the ring pattern parameter for the outer diameter ranges from 4.0 (mm) to 10 (mm).

39. The method of one of claims 35 to 38, wherein the ring pattern parameter for the inner diameter ranges from 4.0 (mm) to 9.9 (mm).

40. The method of one of claims 35 to 39, wherein the ring pattern parameter for the starting position is one of the following: IN or OUT.

41. The method of one of claims 35 to 40, wherein the ring pattern parameter for the ring tangential spot separation ranges from 1 (µm) to 30 (µm).

42. The method of one of claims 35 to 41,wherein the ring pattern parameter for the ring radial spot separation ranges from 1 (µm) to 30 (µm).

43. The method of one of claims 35 to 42, wherein the ring pattern parameter for the ring energy ranges from 1 (µJ) to15 (µJ).

44. The method of one of claims 35 to 43, wherein the ring pattern parameter for the number of ring layers ranges from 1 to10.

45. The method of one of claims 35 to 44, wherein the ring pattern parameter for the ring layer separation ranges from 1 (µm) to 30 (µm).

46. The method of claims 35 to 45, wherein the ring pattern parameter for the entry cut energy ranges from 1 (µJ) to 15 (µJ).

47. The method of one of claims 35 to 46, wherein the ring pattern parameter for the entry cut length ranges from 0.8 (mm) to 1.5 (mm).

48. The method of one of claims 35 to 47, wherein the ring pattern parameter for the entry cut thickness ranges from 0 (µm) to 50 (µm).

49. The method of one of claims 35 to 48, wherein the ring pattern parameter for the incision axis ranges from 0 (deg) to 359 (deg).

50. The method of one of claims 35 to 49, wherein the ring pattern parameter for the entry cut spot separation ranges from 1 (µm) to 30 (µm).

51. The method of one of claims 35 to 50, wherein the ring pattern parameter for the entry cut line separation ranges from 1 (µm) to 50 (µm).

52. The method of one of claims 35 to 51, wherein the ring pattern parameter for the entry cut layer separation ranges from 1 (µm) to 50 (µm).

53. The method of one of claims 35 to 52, wherein the ring pattern parameter for the depth in contact glass ranges from 0 to 300 (µm).

54. The method of one of claims 35 to 53, wherein the ring pattern parameter for the vertical entry cut overlap ranges from 0 (µm) to 100 (µm).

55. The method of one of claims 35 to 54, wherein the ring pattern parameter for the Z-radius ranges from 0 (mm) to 50 (mm).

56. The method of one of claims 35 to 55, further comprising directing said laser beam to photodisrupt a portion of said target to create a vertical resection for an entry cut into said ring-shaped resection.

57. The method of one of claims 1 to 56, further comprising processing initialization data stored on a computer medium, wherein said initialization data contains operating information relating to said laser system.

58. The method of one of claims 1 to 57, wherein the directing step includes directing an optical train of pulses with a duration of approximately 600 femtoseconds at a repetition rate of up to 10,000 Hz.

59. The method of one of claims 1 to 58, further comprising homing galvanometers after the completion of a procedure.

60. The method of one of claims 1 to 59, further comprising validating the values of the different pattern parameters relative to each other to ensure every point of the pattern remains within an optical zone.

61. The method of one of claims 1 to 60, further comprising generating X, Y, and Z values for positioning X, Y and Z galvanometers for focusing said laser beam for photodisruption.
